# FASCICULE DE BREVET EUROPEEN

(11) **EP 1 345 580 B1**
(45) Date de publication et mention de la délivrance du brevet: **07.06.2006**
(21) Numéro de dépôt: 01272054.6
(22) Date de dépôt: 26.11.2001
(51) Int. Cl.: A61Q 5/10, A61K 8/35, A61K 8/41

(54) **COMPOSITION POUR LA TEINTURE D'OXYDATION DES FIBRES KERATINIQUES COMPRENANT UN DIAMINO PYRAZOLE ET UN COMPOSE CARBONYLE**
OXIDATIONSHAARFÄRBEMITTEL ENTHALTEND EIN DIAMINOPYRAZOL UND EINE CARBONYL-VERBINDUNG
COMPOSITION FOR OXIDATION DYEING OF KERATINOUS FIBRES COMPRISING A DIAMINO PYRAZOLE AND A CARBONYL COMPOUND

(30) Priorité: 22.12.2000 FR 0016952
(43) Date de publication de la demande: 24.09.2003
(73) Titulaire: L'OREAL, 75008 Paris (FR)
(72) Inventeur: COTTERET, Jean, F-78480 Verneuil sur Seine (FR)
(74) Mandataire: Fevrier, Murielle Françoise E.
(86) Numéro de dépôt international: PCT/FR2001/003729
(87) Numéro de publication internationale: WO 2002/051373

(56) Documents cités:
- EP-A- 0 873 745
- WO-A-00/38640
- DE-A- 3 843 892
- DE-A- 4 234 886
- US-A- 5 718 731

## Description

La présente invention a pour objet une composition pour la teinture d'oxydation des fibres kératiniques, en particulier des fibres kératiniques humaines telles que les cheveux, comprenant au moins une base d'oxydation choisie parmi les 4,5 ou 3,4-diamino pyrazoles et les triamino pyrazoles, en association avec au moins un composé carbonylé sélectionné, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être choisis notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892, DE 4 234 887, DE 4 234 886, DE 4 234 885 ou DE 195 43 988 des compositions pour la teinture d'oxydation des fibres kératiniques contenant à titre de base d'oxydation des dérivés de pyrazole tels que des 4,5-diamino pyrazoles, des 3,4-diamino pyrazoles ou des 3,4,5-triamino pyrazoles, De telles compositions ne sont cependant pas entièrement satisfaisantes car lors des processus de coloration se produisent des réactions secondaires qui peuvent avoir des effets négatifs du point de vue de l'innocuité et des propriétés des colorations obtenues et notamment de la puissance et de la ténacité des colorations vis à vis des diverses agressions que peuvent subir les cheveux.

Le brevet américain US 5,718,731 décrit une composition de teinture contenant une basse d'oxydation du type 4,5-diaminopyrazole et un dérivé carbonylé choisi parmi N-[(3-diméthylamino)-phenyl]urée, le N-(3-amino-4-méthoxy-phényl) urée et le N-(3-amino-4-méthyl-phényl) urée.

L'invention a pour but de développer de nouvelles compositions tinctoriales ne présentant pas les inconvénients des teintures de la technique antérieure, en particulier, des teintures puissantes, particulièrement résistantes aux diverses agressions que peuvent subir les cheveux et présentant une bonne innocuité.

A cet effet, l'invention a pour objet une composition pour la teinture d'oxydation des fibres kératiniques humaines et en particulier des fibres kératiniques humaines telles que les cheveux, comprenant dans un milieu approprié pour la teinture :
- au moins une base d'oxydation choisie parmi les 4,5 ou 3,4-diamino pyrazoles et les triamino pyrazoles,
- au moins un composé carbonylé sélectionné parmi les composés de formules (I), (II), (III), et les polylmides 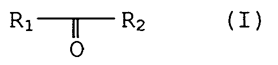
dans laquelle
- R₁ et R₂ désignant indépendamment l'un de l'autre un atome d'hydrogène; une chaîne hydrocarbonée aliphatique, saturée ou insaturée, comportant de 1 à 30 atomes de carbone, Interrompus ou non par un ou plusieurs hétéroatomes ou par un ou plusieurs groupements carbonyle, non substituée ou substituée par un ou plusieurs groupements choisis parmi les groupements hydroxy, alcoxy en C₁-C₄, amino, carboxy, alcoxycarbonyle en C₁-C₁₀, halogène, nitro, mono- ou di-alkyl(C₁-C₄)amino, mono ou dihydroxyalkyl(C₁-C₄)amino ou aryle en C₈-C₂₀ ; un groupement aryle en C₆-C₂₀ non substitué ou substitué par un ou plusieurs groupements choisis parmi les groupements hydroxy, amino, nitro, halogène, carboxy, alcoxycarbonyle(C₁-C₁₀), alkyle(C₁-C₄), mono ou polyhydroxyalkyle(C₁-C₄),
alcoxy(C₁-C₄), mono- ou di-alkyl(C₁-C₄)amino, mono ou dihydroxyalkyl(C₁₋C₄)amino.
R₁ et R₂ peuvent former ensemble avec le groupement C=O un cycle saturé, éventuellement accolé à un ou plusieurs noyaux benzéniques pouvant être substitués par un ou plusieurs radicaux alkyle en C₁-C₁₀, alcoxy en C₁-C₁₀, carboxy ou alcoxycarbonyle en C₁-C₁₀, 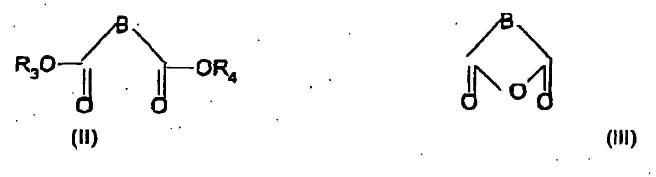 dans lesquelles :
- B désigne une chaîne hydrocarbonés aliphatique, saturée ou insaturée, comportant de 1 à 30 atomes de carbone pouvant être interrompue par un ou plusieurs hétéroatomes ou par un ou plusieurs groupements carbonyle, non substituée ou substituée par un ou plusieurs groupements choisis parmi les groupements hydroxy, alcoxy en C₁-C₄, amino, carboxy, alcoxycarbonyle(C₁-C₁₀), halogène, nitro, mono- ou di-alkyl(C₁-C₄)amino, mono ou dihydroxyalkyl(C₁₋C₄)amino ou aryle en C₆-C₂₀: un groupement aryle en C₆-C₂₀ non substitué ou substitué par un ou plusieurs groupements choisis parmi les groupements hydroxy, amino ,nitro, halogène, carboxy, alcoxycarbonyle(C₁-C₁₀), alkyle(C₁-C₄), mono ou poly hydroxyalkyle(C₁-C₄), alcoxy(C₁-C₄), mono- ou di-alkyl(C₁-C₄)amino, mono ou dihydroxyalkyl(C₁-C₄)amino,
- R₃ et R₄, Identiques ou différents, représentent un atome d'hydrogène, un radical alkyle en C₁-C₁₀, rnonohydroxyaltkyle en C₁-C₁₀; polyhydroxyalkyle en C₂-C₁₀. Na, K ou NH₄.

La composition de teinture d'oxydation de l'invention permet d'obtenir avec une bonne innocuité des colorations puissantes aux nuances variées, peu sélectives et présentant d'excellentes propriétés de résistance à la fois vis à vis des agents atmosphériques tels que la lumière et les Intempéries et vis à vis de la transpiration et des différents traitements que peuvent subir les cheveux (shampooings, déformations permanentes).

Sauf, indication contraire, tous les radicaux, substituants, groupements chaines dans le cadre de l'invention sont linéaires ou ramifiés, substitués ou non.

Parmi les composés de formula (I) ci dessus, on peut citer les aldéhydes ou cétones aliphatiques comme l'acétone, les aldéhydes ou cétones aromatiques comme le p.diméthylaminobenzaldéhyde ou le 5-nitrosalicylaldéhyde, les composés β-dicarbonylés comme l'αα-diméthylacétylacétone, les γ-pyrones tel que la 2.6-diméthyl pyrone, la 2,6 di(éthoxycarbonyl)pyrone, la 2-hydroxy 6-méthyl pyrone, les chromanes comme la 2-méthyl chromone, les aldoses ou cétoses tels que le glycéraldéhyde, la dihydroxyacétone, le D-glucose, le D-fructose, le D-érythrose, le D-ribose, le D-xylose, le D-thréose,le D-érythrulose et le D-sorbose.

Parmi les composés de formule (II) ci dessus, on peut citer l'acide maléique.

Parmi les composés de formule (III) et dessus, on peut citer l'anhydride maléique.

Les polyimides sont des polycondensats dérivés de tetraacides (ou de dianhydrides) et de diamines. De préférence parmi ceux ci on utilise les polyimides aromatiques. On peut citer par exemple les polyimides obtenus à partir du dianhydride pyromellitique ou de la benzophénone dianhydride. On peut citer tout particulièrement les produits issus du dianhydride pyromellitique et de 4,4'-diamino diphényléther (Kapton H de la société duPONT)

Parmi les composés carbonylés de l'invention, on préfère utiliser l'urée, les cétones aliphatiques ou aromatiques, l'acide maléique, les cétoses ou les aldoses.

Le ou les composés carbonylés conformes à l'invention représentent de préférence de 0.00001 à 10 % en poids environ du poids total de la composition tinctoriale, encore plus préférentiellement de 0,001 à 5 % et encore plus préférentiellement de 0,001 à 3 % en poids environ de ce poids.

Parmi les 4,5 ou 3,4-diamino pyrazoles utiles dans les compositions tinctoriales de l'invention, on peut particulièrement citer les diamino pyrazoles choisis parmi les 4,5 ou 3,4-diamino pyrazoles de formules (IV) ou (V) suivantes et/ou leurs sels d'addition avec un acide: 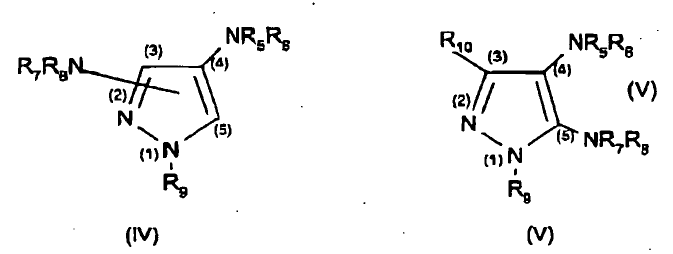 dans laquelle:
- R₅, R₆, R₇, R₈ et R₉, identiques ou différents, représentent un atome d'hydrogène ; un radical alkyle en C₁-C₆ non substitué ou substitué par au moins un substituant choisi parmi OR, NHR, NRR', SR, SOR, SO₂R, COR, COOH, CONH₂, CONHR, CONRR', PO(OH)₂, SH, SO₃X, un hétérocycle non cationique, Cl, Br ou I, X désignant un atome d'hydrogène, Na, K, ou NH₄, et R et R', identique ou différents, représentant un alkyle ou alcényle en C₁-C₄ ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; un radical 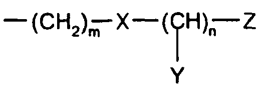 dans lequel m et n sont des nombres entiers, identiques ou différents, compris entre 0 et 3 inclusivement, X représente un atome d'oxygène ou bien le groupement NH, Y représente un atome d'hydrogène ou bien un radical alkyle en C₁-C₄, et Z représente un radical méthyle lorsque n est égal à 0, ou Z représente un radical alkyle en C₁-C₄, un groupement OR ou NR"R"' lorsque n est supérieur ou égal à 1, R" et R"', identiques ou différent, désignant un atome d'hydrogène, un radical alkyle en C₁-C₄ ; ou R₉ forme avec l'atome d'azote du groupement NR₇R₈ en position 5 un hétérocycle comprenant au moins 4 chaînons,
- R₁₀ représente un radical alkyle en C₁-C₆ ; un radical hydroxyalkyle en C₁-C₄ ; un radical aminoalkyle en C₁-C₄ ; un radical alkyle(C₁-C₄)aminoalkyle en C₁-C₄ ; un radical dialkyle (C₁-C₄)-aminoalkyle en C₁-C₄ ; un radical hydroxyalkyle (C₁-C₄)-aminoalkyle en C₁-C₄ ; un radical alcoxy(C₁-C₄)méthyle ; un radical phényle ; un radical phényle substitué par un atome d'halogène ou par un radical alkyle(C₁-C₄), alcoxy(C₁-C₄), nitro, trifluorométhyle, amino ou alkyl(C₁-C₄)amino ; un radical benzyle ; un radical benzyle substitué par un atome d'halogène ou par un radical alkyle(C₁-C₄), alcoxy(C₁-C₄), nitro, trifluorométhyle, amino ou alkyl(C₁-C₄)amino; un hétérocycle choisi parmi le thiophène, le furane et la pyridine, ou encore un radical -(CH₂)_{P}-O-(CH₂)_{q}-OR", dans lequel p et q sont des nombres entiers, identiques ou différents, compris entre 1 et 3 inclusivement et R" est tel que défini précédemment,
étant entendu que :
- au moins un des radicaux R₅, R₆, R₇ et R₈ représente un atome d'hydrogène.

Parmi les triamino pyrazoles utiles à titre de base d'oxydation dans les compositions tinctoriales conformes à l'invention, on peut plus particulièrement citer les composés de formule (VI) suivante, et leurs sels d'addition avec un acide : 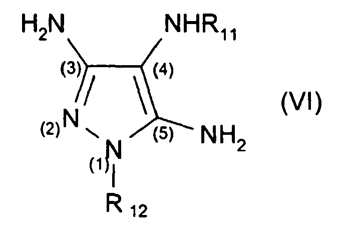 dans laquelle :
- R₁₁ et R₁₂, identiques ou différents, représentent un atome d'hydrogène, un radical alkyle en C₁-C₄ ou hydroxyalkyle en C₂-C₄.

Parmi les 4,5 ou 3,4-diamino pyrazoles de formule (IV) ci-dessus, on peut plus particulièrement citer le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-éthyl pyrazole, , le 4,5-diamino 1-isopropyl pyrazole,le 4,5-diamino 1-hydroxyéthyl pyrazole, le 4,5-diamino 1-benzyl pyrazole, le 4-diamino 5-hydroxyéthylamino 1-hydroxyéthyl pyrazole, le 4-diamino 5-méthylamino 1-hydroxyéthyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine,l'acide 2-(4,5-diamino-pyrazol-1-yl)- 1-éthane sulfonique, le 2(4,5-diamino-pyrazol-1-yl)-acétamide, l'acide 2-(4,5-diamino-pyrazol-1-yl)-acétique, la 2-(2-diméthylamino-éthyl)-2H-pyrazole-3,4-diamine, la 2-(2-méthoxy-éthyl)-2H-pyrazole-3,4-diamine et leurs sels d'addition avec un acide.

Les diaminopyrazoles utile dans la présente invention peuvent être obtenus par des procédés de synthèses bien connus de l'homme du métier. Par exemple, les 4,5-diamino pyrazoles de formule (V) peuvent être préparés selon le procédé de synthèse tel que décrit par exemple dans la demande de brevet français FR-A-2 733 749.

Parmi les 4,5-diamino pyrazoles de formule (V) ci-dessus, on peut plus particulièrement citer :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényi) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

Parmi les 4,5 ou 3,4-diamino pyrazoles de formule (IV) ci-dessus, on préfère plus particulièrement :
- le 4,5-diamino 1- benzyle pyrazole
- le 4,5-diamino 1-(4'-chlorobenzyle) pyrazole
- le 4,5-diamino 1-méthyl pyrazole
- le 4,5-diamino 1-hydroxyéthyl pyrazole
- la 2-(2-méthoxy-éthyl)-2H-pyrazole-3,4-diamine
et leurs sels d'addition avec un acide.

Parmi les 4,5-diamino pyrazoles de formule (V) ci-dessus, on préfère plus particulièrement :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

Parmi les triamino pyrazoles de formule (VI) ci-dessus, on peut plus particulièrement citer le 3,4,5-triamino pyrazole, le 1-méthyl 3,4,5-triamino pyrazole, le 3,5-diamino, 1-méthyl 4-méthylamino pyrazole, et le 3,5-diamino 4-(β-hydroxyéthyl)amino 1-méthyl pyrazole, et leurs sels d'addition avec un acide.

Le ou les 4,5 ou 3,4-diamino pyrazoles et/ou le ou les triamino pyrazoles conformes à l'invention et/ou le ou les sels d'addition avec un acide correspondants représentent de préférence de 0,0005 à 12 % en poids environ du poids total de la composition tinctoriale, et encore plus préférentiellement de 0,005 à 6 % en poids environ de ce poids.

De préférence, le rapport pondéral du ou des composés carbonylés avec le ou les 4,5 ou 3,4-diamino pyrazoles et/ou le ou les triamino pyrazoles et/ou le ou les sels d'addition avec un acide est compris entre 0,001 et 100 et encore plus préférentiellement entre 0,01 et 10.

Les compositions tinctoriales conformes à l'invention contiennent de préférence au moins un coupleur. Les coupleurs utilisables sont ceux classiquement utilisés pour la teinture d'oxydation et notamment les métaphénylènediamines, les métaaminophénols, les métadiphénols, les dérivés de naphtol et les coupleurs hétérocycliques.

Les méta-phénylènediamines, les méta-aminophénols et les méta-diphénols pouvant être utilisés à titre de coupleurs additionnels dans la composition tinctoriale conforme à l'invention sont de préférence choisis parmi les composés répondant à la formule (1) suivante et leurs sels d'addition avec un acide : 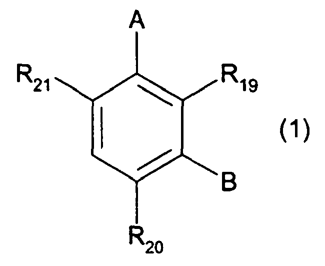 dans laquelle :
- A et B, identiques ou différents, représentent un radical hydroxyle, amino ou -NHR₂₂ dans lequel R₂₂ représente un radical alkyle en C₁-C₄, monohydroxyalkyle en C₁-C₄, ou polyhydroxyalkyle en C₂-C₄,
- R₁₉, R₂₀ et R₂₁, identiques ou différents, représentent un atome d'hydrogène ou d'halogène tel qu'un atome de brome, de chlore, d'iode ou de fluor, un radical alkyle en C₁-C₄, alcoxy en C₁-C₄, monohydroxyalkyle en C₁-C₄ ou polyhydroxyalkyle en C₂-C₄, monohydroxyalcoxy en C₁-C₄ ou polyhydroxyalcoxy en C₂-C₄.

Parmi les composés de formule (1) ci-dessus, on peut notamment citer le 2-méthyl 5-amino phénol, le 2-méthyl 5-amino 6-chloro phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 2,6bis(β-hydroxyéthylamino) toluène et leurs sels d'addition avec un acide.

Le ou les coupleurs hétérocycliques pouvant être utilisés à titre de coupleurs additionnels dans la composition tinctoriale conforme à l'invention peuvent notamment être choisis parmi les dérivés indoliques, les dérivés indoliniques, les dérivés pyridiniques, les dérivés pyrimidiniques, les pyrazolones, et leurs sels d'addition avec un acide. Parmi ces coupleurs hétérocycliques, on peut en particulier citer à titre d'exemple, le sésamol, le 1-N(β-hydroxyéthyl)amino 3'4-méthylènedioxy benzène, le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, la 3,5-diamino 2,6-diméthoxy pyridine, la 2-amino 3-hydroxy pyridine le 1-H 3-méthyl pyrazole 5-one, le 1-phényl 3-méthyl pyrazole 5-one, et leurs sels d'addition avec un acide. Parmi les dérivés de naphtol on peut citer l'α-naphtol, le 2-méthyl-1-naphtol.

Le ou les coupleurs additionnels représentent de préférence de 0,0001 à 10 % en poids environ du poids total de la composition tinctoriale et encore plus préférentiellement de 0,005 à 5 % en poids environ de ce poids.

Les compositions tinctoriales conformes à l'invention peuvent également contenir d'autres bases d'oxydation classiquement utilisées pour la teinture d'oxydation, différentes d'un diamino pyrazole et d'un triamino pyrazole et/ou des colorants directs notamment pour modifier les nuances ou les enrichir en reflets.

Les bases d'oxydation additionnelles utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques autres que les pyrazoles de l'invention ainsi que leurs sels d'addition avec un acide, et notamment :
- **(I)** les paraphénylènediamines de formule (2) suivante et leurs sels d'addition avec un acide : 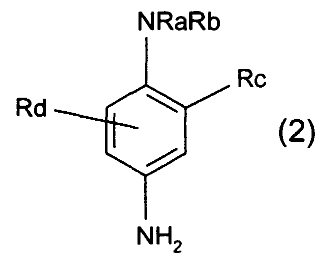 dans laquelle :
   Rₐ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   **R**_{**b**} représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   **R**_{**c**} représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   **R**_{**d**} représente un atome d'hydrogène, d'halogène ou un radical alkyle en C₁-C₄.
   **R**_{**a**} et **R**_{**b**} peuvent également former avec l'atome d'hydrogène qui les porte un hétérocycle azoté à 5 ou 6 chaînons éventuellement substitué par un ou plusieurs groupements alkyle, hydroxy ou uréido.

   Parmi les groupements azotés de la formule (2) ci-dessus, on peut citer notamment les radicaux amino, monoalkyl(C₁-C₄)amino, dialkyl(C₁-C₄)amino, trialkyl(C₁₋C₄)amino, monohydroxyalkyl(C₁-C₄)amino, imidazolinium et ammonium.
   Parmi les paraphénylènediamines de formule (2) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(P-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide.
   Parmi les paraphénylènediamines de formule (2) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** les bases doubles sont des composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (3) suivante, et leurs sels d'addition avec un acide : 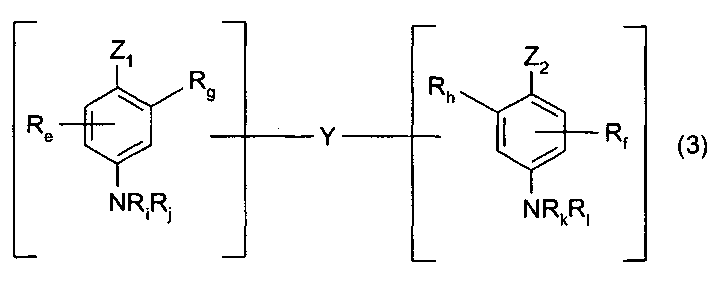 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - Rₑ et R_{f} représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R_{g}, Rₕ, Rᵢ, Rⱼ, Rₖ et Rₗ, identiques ou différents, représentent un atome d'hydrogène, un bras de liaison Y ou un radical alkyle en C₁-C₄ ;
   étant entendu que les composés de formule (3) ne comportent qu'un seul bras de liaison Y par molécule.
   Parmi les groupements azotés de la formule (3) ci-dessus, on peut citer notamment les radicaux amino, monoalkyl(C₁-C₄)amino, dialkyl(C₁-C₄)amino, trialkyl(C₁₋C₄)amino, monohydroxyalkyl(C₁-C₄)amino, imidazolinium et ammonium.
   Parmi les bases doubles de formules (3) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,6-dioxaoctane, et leurs sels d'addition avec un acide. Parmi ces bases doubles de formule (3), le N,N'-bis-(β-hydroxyéthyl) N,N'-bis-(4'-aminophényl) 1,3-diamino propanol, le 1,8-bis-(2,5-diaminophénoxy)-3,6-dioxaoctane ou l'un de leurs sels d'addition avec un acide sont particulièrement préférés.
- **(III)** les para-aminophénols répondant à la formule (4) suivante, et leurs sels d'addition avec un acide : 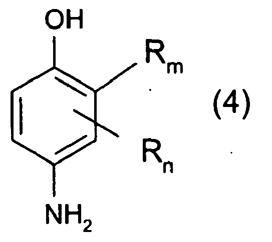 dans laquelle :
   **R**_{**m**} représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R**_{**n**} représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (4) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl-pyrazolo-[1,5-a]-pyrimidine-3,7-diamine, la 2,6-diméthyl-pyrazolo-[1,5-a]-pyrimidine-3,7-diamine, la 2, 5, N7, N7-tetraméthyl-pyrazolo-[1,5-a]-pyrimidine-3,7-diamine, et leurs sels d'addition et leurs formes tautomères, lorsqu'il existe un équilibre tautomérique et leurs sels d'addition avec un acide.

Selon la présente invention, les bases d'oxydation additionnelles peuvent représenter de préférence de 0,0005 à 12% en poids environ du poids total de la composition tinctoriale

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. II peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (5) suivante : 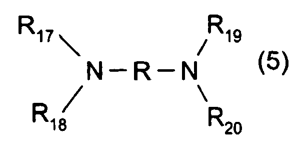 dans laquelle R est un reste propylène éventuellement substitué par un groupement hydroxyle ou un radical alkyle en C₁-C₄ ; R₁₇, R₁₈, R₁₉ et R₂₀, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un premier procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant, cet agent oxydant pouvant être ajouté juste au moment de l'emploi à la composition tinctoriale ou au moyen d'une composition oxydante appliquée simultanément ou séquentiellement.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 60 minutes environ, de préférence 5 à 40 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, percarbonates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition tinctoriale qui est appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Selon une variante, on applique sur ces fibres dans un premier temps une composition contenant au moins le composé carbonylé, et dans un deuxième temps une composition contenant au moins un diamino-pyrazole, l'application de la composition contenant le ou les composés carbonylés étant ou non suivie par une étape de rinçage, la couleur étant révélée à l'aide d'un agent oxydant

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Selon un mode de réalisation différent, le dispositif comprend au moins trois compartiments, un premier compartiment qui contient le composé carbonylé utile pour l'invention, un deuxième compartiment qui contient un diaminopyrazole, et un troisième compartiment qui contient une composition oxydante.

Les exemples qui suivent sont destinés à illustrer l'invention sans pour autant en limiter la portée.

### EXEMPLES

### EXEMPLES DE TEINTURE 1 à 9

On a préparé les compositions tinctoriales, conformes à l'invention, suivantes (teneurs en grammes) :

**M.A. désigne Matière Active**

| **EXEMPLES** | **1** | **2** | **3** | **4** | **5** | **6** | **7** | **8** | **9** |
|---|---|---|---|---|---|---|---|---|---|
| 4,5-diamino 1-βhydroxyéthyl pyrazole, 2 HCl (base d'oxydation) | 0,645 | 0.645 | 0,645 | 0,645 | 0,645 | 0,645 | 0,645 | 0,645 | 0,645 |
| 3-amino 6-méthyl phénol (coupleur) | 0,369 | 0,369 | 0,369 | 0,369 | 0,369 | 0,369 | 0,369 | 0,369 | 0.369 |
| p.diméthylamino benzaidéhyde (composé carbonylé selon l'invention) | 0.05 | - | - | - | - | - | - | - | - |
| 2-hydroxy 6-méthyl pyrone (composé carbonylé selon l'invention) | - | 0,15 | - | - | - | - | - | - | - |
| 2-méthyl chromone (composé carbonylé selon l'invention) | - | - | 0.2 | - | - | - | - | - | - |
| D-fructose | - | - | - | 0,5 | - | - | - | - | - |
| Acide maléique (composé carbonylé selon l'invention) | - | - | - | - | 0,5 | - | - | - | - |
| Anhydride maléique (composé carbonylé selon l'invention) | - | - | - | - | - | 0,4 | - | - | - |
| Kapton H (DuPONT) | - | - | - | - | - | - | 1MA | - | - |
| Acétone (composé carbonylé selon l'invention) | - | - | - | - | - | - | - | 0,1 | - |
| ααdiméthylacétyl acétone (composé carbonylé selon l'inventicin) | - | - | - | - | - | - | - | - | 0,2 |
| Support de teinture commun | (**) | (**) | (**) | (**) | (**) | (**) | (**) | (**) | (**) |
| Eau déminéralisée q.s.p. | 100 g | 100 g | 100 g | 100 g | 100 g | 100 g | 100 g | 100 g | 100 g |

| | | | | | | | | | |
|---|---|---|---|---|---|---|---|---|---|
| (**) support de teinture commun contenant : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3.0 g - Amine oléique à 2 moles d'oxyde d'éthylène vendue sous la dénomination commerciale ETHOMEEN O12 par la société AKZO 7.0 g - Laurylamino succinamate de diéthylaminopropyle, sel de sodium, à 55% de M.A. 3.0 g M.A. - Alcool oléique 5,0 g - Diéthanolamide d'acide oléique 12,0 g - Propylèneglycol 3.5 g - Alcool éthylique 7,0 g - Dipropylèneglycol 0,5 g - Monométhyléther de propylèneglycol 9,0 g - Métabisulfite de sodium en solution aqueuse, à 35 % de M.A 0.455 g M.A. - Acétate d'ammonium 0,8 g - Antioxydant, séquestrant q.s. - Parfum, conservateur q.s. -Ammoniaque à 20 % de NH₃ 10 g | | | | | | | | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

On obtient dans tous les cas une nuance rouge puissante et tenace.avec une bonne innoculté.

### EXEMPLES DE TEINTURE 10 à 11

On a préparé les compositions tinctoriales, conformes à l'invention, suivantes (teneurs en grammes) :

| **EXEMPLE** | **10** | **11** |
|---|---|---|
| 4,5-diamino 1-éthyl 3-méthyl pyrazole, 2 HCl (base d'oxydation) | 0,639 | - |
| 3,4,5-triamino pyrazole, 2 HCl (base d'oxydation) | - | 0,667 |
| 3-amino 6-méthyl phénol (coupleur) | 0,369 | 0,369 |
| D-fructose (composé carbonylé selon l'invention) | 0,5 | - |
| Acide maléique (composé carbonylé selon l'invention) | - | 0,5 |
| Support de teinture commun | (**) | (**) |
| Eau déminéralisée q.s.p. | 100 g | 100 g |

| | | |
|---|---|---|
| (**) support de teinture commun : identique à celui des exemples 1 à 10 | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

On obtient dans les trois exemples une nuance puissante et tenace avec une bonne innocuité.

## Revendications

1. Composition pour la teinture d'oxydation des fibres kératiniques humaines et en particulier des fibres kératiniques humaines telles que les cheveux, comprenant dans un milieu approprié pour la teinture :
- au moins une base d'oxydation choisie parmi les 4,5 ou 3,4-diamino pyrazoles et les triamino pyrazoles,
- au moins un composé carbonylé sélectionné parmi les composés de formules (I), (II), (III), et les polyimides 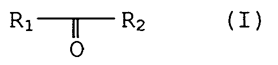 dans laquelle
- R₁ et R₂ désignent indépendamment l'un de l'autre un atome d'hydrogène; une chaîne hydrocarbonée, aliphatique, saturée ou Insaturée, comportant de 1 à 30 atomes de carbone, interrompue ou non par un ou plusieurs hétéroatomes ou par un ou plusieurs groupements carbonyle, non substituée ou substituée par un ou plusieurs groupements choisis parmi les groupements hydroxy, alcoxy en C₁-C₄, amino, carboxy, alcoxycarbonyle en C₁-C₁₀, halogène, nitro, mono-
ou di-alkyl(C₁-C₄)amino, mono ou dihydroxyalkyl(C₁-C₄)amino ou aryle en C₈-C₂₀ ; un groupement aryle en C₈-C₂₀ non substitué ou substitué par un ou plusieurs groupements choisis parmi les groupements hydroxy, amino, nitro, halogène, carboxy, alcoxyrarbonyle(C₁-C₁₀), alkyle(C₁-C₄), mono ou polyhydroxyalkyle(C₁-C₄), alcoxy(C₁-C₄), mono- ou di-alkyl(C₁-C₄)amino , mono ou dihydroxyalkyl(C₁₋C₄)amino.
R₁ et R₂ peuvent former ensemble avec le groupement C=O un cycle saturé, éventuellement accolé à un ou plusieurs noyaux benzéniques pouvant être substitués par un ou plusieurs radicaux alkyle en C₁-C₁₀, alcoxy en C₁-C₁₀, carboxy ou alcoxycarbonyle en C₁-C₁₀, 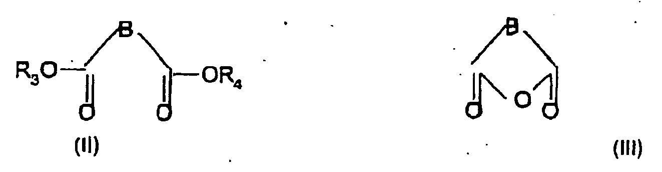
dans lesquelles :
- B désigne une chaîne hydrocarbonée aliphatique, saturée ou insaturée, comportant de 1 à 30 atomes de carbone, pouvant être interrompue par un ou plusieurs hétéroatomes ou par un ou plusieurs groupements carbonyle, non substituée ou substituée par un ou plusieurs groupements choisis parmi les groupements hydroxy, alcoxy en C₁-C₄, amino, carboxy, alcoxycarbonyle(C₁-C₁₀), halogène, nitro, mono- ou di-alkyl(C₁-C₄)amino, mono ou dihydroxyalkyl(C₁₋C₄)amino ou aryle en C₈-C₂₀ : un groupement aryle en C₈-C₂₀ non substitué ou substitué par un ou plusieurs groupements choisis parmi les groupements hydroxy, amino nitro, halogène, carboxy, alcoxycarbonyle(C₁-C₁₀), alkyle(C₁-C₄), mono ou poly hydroxyalkyle (C₁-C₄), alcoxy (C₁-C₄), mono- ou di-alkyl (C₁-C₄)amino, mono ou dihydroxyalkyl(C₁-C₄)amino,
- R₃ et R₄, identiques ou différents, représentent un atome d'hydrogène, un radical alkyle en C₁-C₁₀, monohydroxyalkyle en C₁-C₁₀ polyhydroxyalkyle en C₂-C₁₀, Na, K ou NH₄.

2. Composition selon la revendication 1, **caractérisée en ce que** les composés de formule (I) sont choisis parmi les aldéhydes ou cétones aliphatiques, les aldéhydes ou cétones aromatiques, les composés β-dicarbonylés , les γ-pyrones, les chromones, les aldoses ou cétoses.

3. Composition selon la revendication 1, **caractérisée en ce que** le composé de formule (II) est l'acide maléique.

4. Composition selon la revendication 1, **caractérisée en ce que** le composé de formule (III) est l'anhydride maléique.

5. Composition selon la revendication 1, **caractérisée en ce que** le polyimide est un polyimide aromatique.

6. Composition selon la revendication 5, **caractérisée en ce que** le polyimide est obtenu à partir du dianhydride pyroméllitique ou de la benzophénone dianhydride.

7. Composition selon l'une quelconque des revendications 1 à 6, **caractérisée en ce que** le ou les composés carbonylés représentent de préférence de 0,00001 à 10 % en poids environ du poids total de la composition tinctoriale, encore plus préférentiellement de 0,001 à 5 % et encore plus préférentiellement de 0,001 à 3 % en poids environ de ce poids.

8. Composition selon l'une quelconque des revendications 1 à 7, **caractérisée en ce que** les 4,5 ou 3,4-diamino pyrazoles sont choisis parmi les 4,5 ou 3,4-diamino pyrazoles de formules (IV) ou (V) suivantes et/ou leurs sels d'addition avec un acide : 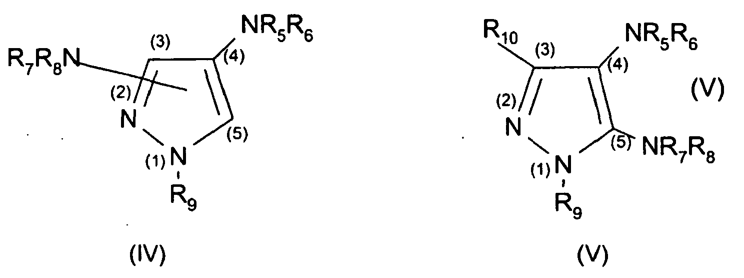 dans laquelle :
- R₅, R₆, R₇, R₈ et R₉, identiques ou différents, représentent un atome d'hydrogène ; un radical alkyle en C₁-C₆ non substitué ou substitué par au moins un substituant choisi parmi OR, NHR, NRR', SR, SOR, SO₂R, COR, COOH, CONH₂, CONHR, CONRR', PO(OH)₂, SH, SO₃X, un hétérocycle non cationique, Cl, Br ou l, X désignant un atome d'hydrogène, Na, K, ou NH₄, et R et R', identique ou différents, représentant un alkyle ou alcényle en C₁-C₄ ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; un radical 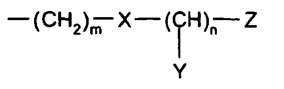 dans lequel m et n sont des nombres entiers, identiques ou différents, compris entre 0 et 3 inclusivement, X représente un atome d'oxygène ou bien le groupement NH, Y représente un atome d'hydrogène ou bien un radical alkyle en C₁-C₄, et Z représente un radical méthyle lorsque n est égal à 0, ou Z représente un radical alkyle en C₁-C₄, un groupement OR ou NR"R"' lorsque n est supérieur ou égal à 1, R" et R"', identiques ou différent, désignant un atome d'hydrogène, un radical alkyle en C₁-C₄ ; ou R₉ forme avec l'atome d'azote du groupement NR₇R₈ en position 5 un hétérocycle comprenant au moins 4 chaînons,
- R₁₀ représente un radical alkyle en C₁-C₆ ; un radical hydroxyalkyle en C₁-C₄ ; un radical aminoalkyle en C₁-C₄ ; un radical alkyle(C₁-C₄)aminoalkyle en C₁-C₄ ; un radical dialkyle (C₁-C₄)-aminoalkyle en C₁-C₄ ; un radical hydroxyalkyle (C₁-C₄)-aminoalkyle en C₁-C₄ ; un radical alcoxy(C₁-C₄)méthyle ; un radical phényle ; un radical phényle substitué par un atome d'halogène ou par un radical alkyle(C₁-C₄), alcoxy(C₁-C₄), nitro, trifluorométhyle, amino ou alkyl(C₁-C₄)amino ; un radical benzyle ; un radical benzyle substitué par un atome d'halogène ou par un radical alkyle(C₁-C₄), alcoxy(C₁-C₄), nitro, trifluorométhyle, amino ou alkyl(C₁-C₄)amino; un hétérocycle choisi parmi le thiophène, le furane et la pyridine, ou encore un radical -(CH₂)ₚ-O-(CH₂)_{q}-OR", dans lequel p et q sont des nombres entiers, identiques ou différents, compris entre 1 et 3 inclusivement et R" est tel que défini précédemment,
étant entendu que :
- au moins un des radicaux R₅, R₆, R₇ et R₈ représente un atome d'hydrogène.

9. Composition selon l'une quelconque des revendications 1 à 7, **caractérisée en ce que** les triamino pyrazoles sont choisis parmi les composés de formule (VI) suivante, et/ou leurs sels d'addition avec un acide : 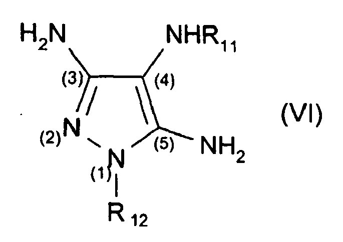 dans laquelle :
- R₁₁ et R₁₂, identiques ou différents, représentent un atome d'hydrogène, un radical alkyle en C₁-C₄ ou hydroxyalkyle en C₂-C₄.

10. Composition selon la revendication 8, **caractérisée en ce que** les 4,5 ou 3,4-diamino pyrazoles de formule (IV) sont choisis parmi le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-éthyl pyrazole, , le 4,5-diamino 1-isopropyl pyrazole,le 4,5-diamino 1-hydroxyéthyl pyrazole, le 4,5-diamino 1-benzyl pyrazole, le 4-diamino 5-hydroxyéthylamino 1-hydroxyéthyl pyrazole, le 4-diamino 5-méthylamino 1-hydroxyéthyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine,l'acide 2-(4,5-diamino-pyrazol-1-yl)- 1-éthane sulfonique, le 2(4,5-diamino-pyrazol-1-yl)-acétamide, l'acide 2-(4,5-diamino-pyrazol-1-yl)-acétique, la 2-(2-diméthylamino-éthyl)-2H-pyrazole-3,4-diamine, la 2-(2-méthoxy-éthyl)-2H-pyrazole-3,4-diamine et leurs sels d'addition avec un acide.

11. Composition selon la revendication 10, **caractérisée par le fait que** les 4,5 ou 3,4-diamino pyrazoles de formule (IV) sont choisis parmi
- le 4,5-diamino 1-benzyle pyrazole
- le 4,5-diamino 1-(4'-chlorobenzyle) pyrazole
- le 4,5-diamino 1-méthyl pyrazole
- le 4,5-diamino 1-hydroxyéthyl pyrazole
- la 2-(2-méthoxy-éthyl)-2H-pyrazole-3,4-diamine
et leurs sels d'addition avec un acide.

12. Composition selon la revendication 8, **caractérisée en ce que** les 4,5-diamino pyrazoles de formule (V) sont choisis parmi:
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino-3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

13. Composition selon la revendication 12, **caractérisée en ce que** les 4,5-diamino pyrazoles de formule (V) sont choisis parmi :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

14. Composition selon la revendication 9, **caractérisée en ce que** les triamino pyrazoles de formule (VI) sont choisis parmi le 3,4,5-triamino pyrazole, le 1-méthyl 3,4,5-triamino pyrazole, le 3,5-diamino, 1-méthyl 4-méthylamino pyrazole, et le 3,5-diamino 4-(β-hydroxyéthyl)amino 1-méthyl pyrazole, et leurs sels d'addition avec un acide.

15. Composition selon l'une quelconque des revendications précédentes 1 à 14, **caractérisée en ce que** le ou les 4,5 ou 3,4-diamino pyrazoles et/ou le ou les triamino pyrazoles et/ou le ou les sels d'addition avec un acide correspondants représentent de 0,0005 à 12 % en poids du poids total de la composition tinctoriale.

16. Composition selon la revendication 15, **caractérisée en ce que** le ou les 4,5 ou 3,4-diamino pyrazoles et/ou le ou les triamino pyrazoles et/ou le ou les sels d'addition avec un acide correspondants représentent de 0,005 à 6 % en poids du poids total de la composition tinctoriale.

17. Composition selon l'une quelconque des revendications précédentes 1 à 16, **caractérisée en ce que** le rapport pondéral du ou des composés carbonylés avec le ou les 4,5 ou 3,4-diamino pyrazoles et/ou le ou les triamino pyrazoles et/ou le ou les sels d'addition avec un acide est compris entre 0,001 et 100 et de préférence entre 0,01 et 10.

18. Composition selon l'une quelconque des revendications précédentes 1 à 17, **caractérisée en ce que** les sels d'addition avec un acide sont choisis parmi les chlorhydrates, les bromhydrates, les sulfates et les tartrates, les lactates et les acétates.

19. Composition selon l'une quelconque des revendications précédentes 1 à 18, **caractérisée en ce qu'**elle contient au moins un coupleur.

20. Composition selon la revendication 19 , **caractérisée en ce que** le ou les coupleurs représentent de 0,0001 à 10% en poids environ du poids total de la composition tinctoriale et encore plus préférentiellement de 0,005 à 5 % en poids environ de ce poids.

21. Composition selon l'une quelconque des revendications précédentes 1 à 20, **caractérisée par le fait qu'**elle contient au moins une base d'oxydation additionnelle autre que les pyrazoles définis dans les revendications 1 à 14.

22. Composition selon la revendication 21 , **caractérisée par le fait que** la ou les bases d'oxydation additionnelles représentent de 0,0005 à 12% en poids environ du poids total de la composition tinctoriale

23. Composition selon l'une quelconque des revendications précédentes 1 à 22, **caractérisée par le fait qu'**elle présente un pH compris entre 3 et 12.

24. Composition selon l'une quelconque des revendications précédentes 1 à 23, **caractérisée en ce qu'**elle se présente sous forme de liquides, de crèmes, de gels, ou sous toute autre forme appropriée pour réaliser une teinture des fibres kératiniques, et notamment des cheveux humains.

25. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 24, et que l'on révèle la couleur à pH acide, neutre ou alcalin à l'aide d'un agent oxydant.

26. Procédé selon la revendication 25, **caractérisé en ce que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides.

27. Procédé de teinture des fibres kératiniques et en particulier des fibres kératiniques humaines telles que les cheveux **caractérisé par le fait que** l'on applique sur ces fibres dans un premier temps une composition contenant au moins un composé carbonylé tel que défini dans l'une quelconque des revendications 1 à 24, dans un deuxième temps une composition contenant au moins un diamino-pyrazole tel que défini dans l'une quelconque des revendications 1 à 24, l'application de la composition contenant le ou les composés carbonylés étant ou non suivie par une étape de rinçage, la couleur étant révélée à l'aide d'un agent oxydant.

28. Dispositif à plusieurs compartiments comprenant un premier compartiment renfermant une composition tinctoriale telle que définie à l'une quelconque des revendications 1 à 24 et un second compartiment renfermant une composition oxydante.

29. Dispositif à plusieurs compartiments comprenant un premier compartiment renfermant un composé carbonylé tel que défini dans l'une quelconque des revendications 1 à 24, un deuxième compartiment renfermant un diaminopyrazole tel que défini dans l'une quelconque des revendications 1 à 24, et un troisième compartiment renfermant une composition oxydante.

## Claims

1. Composition for the oxidation dyeing of human keratin fibres, and in particular of human keratin fibres such as the hair, comprising, in a medium that is suitable for dyeing:
- at least one oxidation base chosen from 4,5- or 3,4-diaminopyrazoles and triaminopyrazoles,
- at least one carbonyl compound selected from the compounds of formulae (I), (II) and (III) and the polyimides 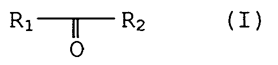 in which
- R₁ and R₂ denote, independently of each other, a hydrogen atom; a saturated or unsaturated aliphatic hydrocarbon-based chain containing from 1 to 30 carbon atoms, which may be interrupted with one or more hetero atoms or with one or more carbonyl groups, which is unsubstituted or substituted with one or more groups chosen from hydroxyl, C₁-C₄ alkoxy, amino, carboxyl, C₁-C₁₀ alkoxycarbonyl, halogen, nitro, mono- or di(C₁-C₉)alkylamino, mono- or dihydroxy(C₁-C₄)alkylamino or C₆-C₂₀ aryl groups; a C₆-C₂₀ aryl group, which is unsubstituted or substituted with one or more groups chosen from hydroxyl, amino, nitro, halogen, carboxyl, (C₁₋C₁₀) alkoxycarbonyl, C₁-C₄ alkyl, mono- or polyhydroxy (C₁-C₄) alkyl, C₁-C₄ alkoxy, mono- or di(C₁-C₄)alkylamino or mono- or dihydroxy(C₁₋C₄)alkylamino groups,
- R₁ and R₂ can form, together with the C=O group, a saturated ring optionally fused to one or more benzene nuclei that may be substituted with one or more C₁-C₁₀ alkyl, C₁-C₁₀ alkoxy, carboxyl or C₁-C₁₀ alkoxycarbonyl radicals, 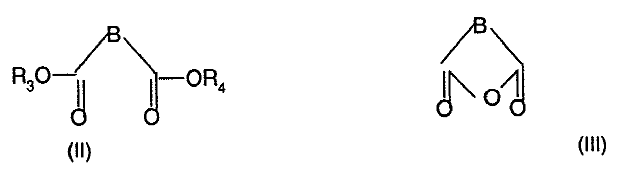
in which:
- B denotes a saturated or unsaturated aliphatic hydrocarbon-based chain containing from 1 to 30 carbon atoms, which may be interrupted with one or more hetero atoms or with one or more carbonyl groups, which is unsubstituted or substituted with one or more groups chosen from hydroxyl, C₁-C₄ alkoxy, amino, carboxyl, C₁-C₁₀ alkoxycarbonyl, halogen, nitro, mono- or di (C₁-C₄) alkylamino, mono- or dihydroxy (C₁-C₄) - alkylamino or C₆-C₂₀ aryl groups; a C₆-C₂₀ aryl group, which is unsubstituted or substituted with one or more groups chosen from hydroxyl, amino, nitro, halogen, carboxyl, (C₁-C₁₀)alkoxycarbonyl, C₁-C₄ alkyl, mono- or polyhydroxy(C₁₋C₄) alkyl, C₁-C₄ alkoxy, mono- or di (C₁₋C₄) alkylamino or mono- or dihydroxy(C₁-C₄)alkylamino groups,
- R₃ and R₄, which may be identical or different, represent a hydrogen atom, a C₁-C₁₀ alkyl, C₁-C₁₀ monohydroxyalkyl or C₂-C₁₀ polyhydroxyalkyl radical, Na, K or NH₄.

2. Composition according to Claim 1, **characterized in that** the compounds of formula (I) are chosen from aliphatic aldehydes or ketones, aromatic aldehydes or ketones, β-dicarbonyl compounds, γ-pyrones, chromones, aldoses and ketoses.

3. Composition according to Claim 1, **characterized in that** the compound of formula (II) is maleic acid.

4. Composition according to Claim 1, **characterized in that** the compound of formula (III) is maleic anhydride.

5. Composition according to Claim 1, **characterized in that** the polyimide is an aromatic polyimide.

6. Composition according to Claim 5, **characterized in that** the polyimide is obtained from pyromellitic dianhydride or benzophenone dianhydride.

7. Composition according to any one of Claims 1 to 6, **characterized in that** the carbonyl compound(s) preferably represent(s) from 0.00001% to 10% by weight approximately relative to the total weight of the dye composition, even more preferentially from 0.001% to 5% and even more preferentially from 0.001% to 3% by weight approximately relative to this weight.

8. Composition according to any one of Claims 1 to 7, **characterized in that** the 4,5- or 3,4-diaminopyrazoles are chosen from the 4,5- or 3,4-diaminopyrazoles of formula (IV) or (V) below, and/or the addition salts thereof with an acid: 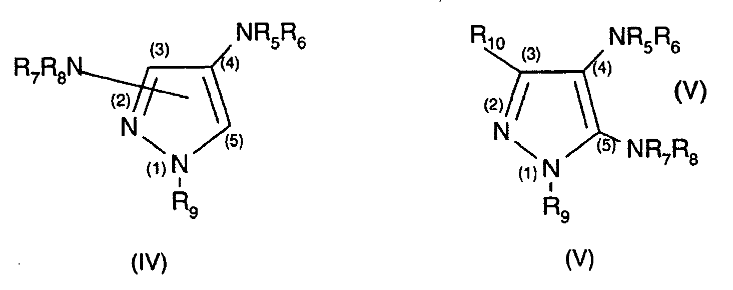 in which:
- R₅, R₆, R₇, R₈ and R₉, which may be identical or different, represent a hydrogen atom; a C₁-C₆ alkyl radical which is unsubstituted or substituted with at least one substituent chosen from OR, NHR, NRR', SR, SOR, SO₂R, COR, COOH, CONH₂, CONHR, CONRR', PO(OH)₂, SH and SO₃X, a non-cationic heterocycle, Cl, Br or I, X denoting a hydrogen atom, Na, K or NH₄, and R and R', which may be identical or different, representing a C₁-C₄ alkyl or alkenyl; a C₂-C₄ hydroxyalkyl radical; a C₂-C₄ aminoalkyl radical; a phenyl radical; a phenyl radical substituted with a halogen atom or a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, methylenedioxy or amino radical; a radical 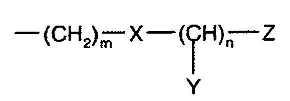 in which m and n are integers, which may be identical or different, between 0 and 3 inclusive, X represents an oxygen atom or an NH group, Y represents a hydrogen atom or a C₁-C₄ alkyl radical, and Z represents a methyl radical when n is equal to 0, or Z represents a C₁-C₄ alkyl radical, a group OR or NR"R"' when n is greater than or equal to 1, R" and R"', which may be identical or different, denoting a hydrogen atom or a C₁-C₄ alkyl radical; or R₉ forms with the nitrogen atom of the group NR₇R₈ in position 5 an at least 4-membered heterocycle,
- R₁₀ represents a C₁-C₆ alkyl radical; a C₁-C₄ hydroxyalkyl radical; a C₁-C₄ aminoalkyl radical; a (C₁-C₄)-alkylamino (C₁-C₄) alkyl radical; a di (C₁-C₄) alkylamino-(C₁-C₉) alkyl radical; a hydroxy (C₁-C₄) alkylamino (C₁-C₉) - alkyl radical; a (C₁-C₄)alkoxymethyl radical; a phenyl radical; a phenyl radical substituted with a halogen atom or with a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, nitro, trifluoromethyl, amino or C₁-C₄ alkylamino radical; a heterocycle chosen from thiophene, furan and pyridine, or a radical -(CH₂)ₚ-O-(CH₂)_{q}-OR", in which p and q are integers, which may be identical or different, between 1 and 3 inclusive and R" is as defined above, it being understood that:
- at least one of the radicals R₅, R₆, R₇ and R₈ represents a hydrogen atom.

9. Composition according to any one of Claims 1 to 7, **characterized in that** the triaminopyrazoles are chosen from the compounds of formula (VI) below, and/or the addition salts thereof with an acid: 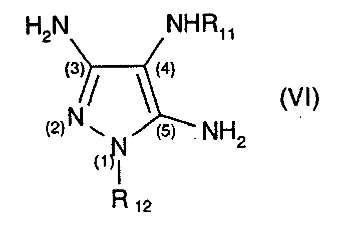 in which:
- R₁₁ and R₁₂, which may be identical or different, represent a hydrogen atom or a C₁-C₄ alkyl or C₂-C₄ hydroxyalkyl radical.

10. Composition according to Claim 8, **characterized in that** the 4,5- or 3,4-diaminopyrazoles of formula (IV) are chosen from 4,5-diamino-1-(4'-methoxybenzyl)-pyrazole, 4,5-diamino-1-(4'-methylbenzyl)pyrazole, 4,5-diamino-1-(4'-chlorobenzyl)pyrazole, 4,5-diamino-1- (3'-methoxybenzyl)pyrazole, 4-amino-1- (4'-methoxybenzyl)-5-methylaminopyrazole, 4-amino-5-(β-hydroxyethyl) amino-1-(4'-methoxybenzyl)pyrazole, 4-amino-5-(β-hydroxyethyl)amino-1-methylpyrazole, 4-amino-(3)-5-methylaminopyrazole, 3-(5),4-diaminopyrazole, 4,5-diamino-1-methylpyrazole, 4,5-diamino-1-ethylpyrazole, 4,5-diamino-1-isopropylpyrazole, 4,5-diamino-1-hydroxyethylpyrazole, 4,5-diamino-1-benzylpyrazole, 4-diamino-5-hydroxyethylamino-1-hydroxyethylpyrazole, 4-diamino-5-methylamino-1-hydroxyethylpyrazole, 3-amino-4,5,7,8-tetrahydropyrazolo[1,5-a]pyrimidine, 7-amino-2,3-dihydro-1H-imidazole[1,2-b]pyrazole, 3-amino-8-methyl-4,5,7,8-tetrahydropyrazolo[1,5-a]-pyrimidine, 2-(4,5-diamino-1-pyrazolyl)-1-ethanesulfonic acid, 2-(4,5-diamino-1-pyrazolyl)acetamide, 2-(4,5-diamino-1-pyrazolyl)acetic acid, 2-(2-dimethylaminoethyl)-2H-pyrazole-3,4-diamine and 2-(2-methoxyethyl)-2H-pyrazole-3,4-diamine, and the addition salts thereof with an acid.

11. Composition according to Claim 10, **characterized in that** the 4,5- or 3,4-diaminopyrazoles of formula (IV) are chosen from:
- 4,5-diamino-1-benzylpyrazole,
- 4,5-diamino-1-(4'-chlorobenzyl)pyrazole,
- 4,5-diamino-1-methylpyrazole,
- 4,5-diamino-1-hydroxyethylpyrazole,
- 2-(2-methoxyethyl)-2H-pyrazole-3,4-diamine,
and the addition salts thereof with an acid.

12. Composition according to Claim 8, **characterized in that** the 4,5-diaminopyrazoles of formula (V) are chosen from:
- 1-benzyl-4,5-diamino-3-methylpyrazole,
- 4,5-diamino-1-(β-hydroxyethyl)-3-(4'-methoxyphenyl)-pyrazole,
- 4,5-diamino-1-(β-hydroxyethyl)-3-(4'-methylphenyl)-pyrazole,
- 4,5-diamino-1-(β-hydroxyethyl)-3-(3'-methylphenyl)-pyrazole,
- 4,5-diamino-3-methyl-1-isopropylpyrazole,
- 4,5-diamino-3-(4'-methoxyphenyl)-1-isopropylpyrazole,
- 4,5-diamino-1-ethyl-3-methylpyrazole,
- 4,5-diamino-1-ethyl-3-(4'-methoxyphenyl)pyrazole,
- 4,5-diamino-3-hydroxymethyl-1-methylpyrazole,
- 4,5-diamino-1-ethyl-3-hydroxymethylpyrazole,
- 4,5-diamino-3-hydroxymethyl-1-isopropylpyrazole,
- 4,5-diamino-3-hydroxymethyl-1-tert-butylpyrazole,
- 4,5-diamino-3-hydroxymethyl-1-phenylpyrazole,
- 4,5-diamino-3-hydroxymethyl-1-(2'-methoxyphenyl)pyrazole,
- 4,5-diamino-3-hydroxymethyl-1-(3'-methoxyphenyl)pyrazole,
- 4,5-diamino-3-hydroxymethyl-1-(4'-methoxyphenyl)pyrazole,
- 1-benzyl-4,5-diamino-3-hydroxymethylpyrazole,
- 4,5-diamino-3-methyl-1-(2'-methoxyphenyl)pyrazole,
- 4,5-diamino-3-methyl-1-(3'-methoxyphenyl)pyrazole,
- 4,5-diamino-3-methyl-1-(4'-methoxyphenyl)pyrazole,
- 3-aminomethyl-4,5-diamino-1-methylpyrazole,
- 3-aminomethyl-4,5-diamino-1-ethylpyrazole,
- 3-aminomethyl-4,5-diamino-1-isopropylpyrazole,
- 3-aminomethyl-4,5-diamino-1-tert-butylpyrazole,
- 4,5-diamino-3-dimethylaminomethyl-1-methylpyrazole,
- 4,5-diamino-3-dimethylaminomethyl-1-ethylpyrazole,
- 4,5-diamino-3-dimethylaminomethyl-1-isopropylpyrazole,
- 4,5-diamino-3-dimethylaminomethyl-1-tert-butylpyrazole,
- 4,5-diamino-3-ethylaminomethyl-1-methylpyrazole,
- 4,5-diamino-3-ethylaminomethyl-1-ethylpyrazole,
- 4,5-diamino-3-ethylaminomethyl-1-isopropylpyrazole,
- 4,5-diamino-3-ethylaminomethyl-1-tert-butylpyrazole,
- 4,5-diamino-3-methylaminomethyl-1-methylpyrazole,
- 4,5-diamino-3-methylaminomethyl-1-isopropylpyrazole,
- 4,5-diamino-1-ethyl-3-methylaminomethylpyrazole,
- 1-tert-butyl-4,5-diamino-3-methylaminomethylpyrazole,
- 4,5-diamino-3-[(β-hydroxyethyl)aminomethyl]-1-methylpyrazole,
- 4,5-diamino-3-[(β-hydroxyethyl)aminomethyl]-1-isopropylpyrazole,
- 4,5-diamino-l-ethyl-3-[(β-hydroxyethyl)aminomethyl]-pyrazole,
- 1-tert-butyl-4,5-diamino-3-[(β-hydroxyethyl)aminomethyl]pyrazole,
- 4-amino-5-(β-hydroxyethyl)amino-1,3-dimethylpyrazole,
- 4-amino-5-(β-hydroxyethyl)amino-1-isopropyl-3-methylpyrazole,
- 4-amino-5-(β-hydroxyethyl)amino-1-ethyl-3-methylpyrazole,
- 4-amino-5-(β-hydroxyethyl)amino-1-tert-butyl-3-methylpyrazole,
- 4-amino-5-(β-hydroxyethyl)amino-1-phenyl-3-methylpyrazole,
- 4-amino-5-(β-hydroxyethyl)amino-1-(2-methoxyphenyl)-3-methylpyrazole,
- 4-amino-5-(β-hydroxyethyl)amino-1-(3-methoxyphenyl)-3-methylpyrazole,
- 4-amino-5-(β-hydroxyethyl)amino-1-(4-methoxyphenyl)-3-methylpyrazole,
- 4-amino-5-(β-hydroxyethyl)amino-1-benzyl-3-methylpyrazole,
- 4-amino-1-ethyl-3-methyl-5-methylaminopyrazole,
- 4-amino-1-tert-butyl-3-methyl-5-methylaminopyrazole,
- 4,5-diamino-1,3-dimethylpyrazole,
- 4,5-diamino-3-tert-butyl-1-methylpyrazole,
- 4,5-diamino-1-tert-butyl-3-methylpyrazole,
- 4,5-diamino-1-methyl-3-phenylpyrazole,
- 4,5-diamino-1-(β-hydroxyethyl)-3-methylpyrazole,
- 4,5-diamino-1-(β-hydroxyethyl)-3-phenylpyrazole,
- 4,5-diamino-1-methyl-3-(2'-chlorophenyl)pyrazole,
- 4,5-diamino-1-methyl-3-(4'-chlorophenyl)pyrazole,
- 4,5-diamino-1-methyl-3-(3'-trifluoromethylphenyl)pyrazole,
- 4,5-diamino-1,3-diphenylpyrazole,
- 4,5-diamino-3-methyl-1-phenylpyrazole,
- 4-amino-1,3-dimethyl-5-phenylaminopyrazole,
- 4-amino-1-ethyl-3-methyl-5-phenylaminopyrazole,
- 4-amino-1,3-dimethyl-5-methylaminopyrazole,
- 4-amino-3-methyl-1-isopropyl-5-methylaminopyrazole,
- 4-amino-3-isobutoxymethyl-1-methyl-5-methylaminopyrazole,
- 4-amino-3-methoxyethoxymethyl-1-methyl-5-methylaminopyrazole,
- 4-amino-3-hydroxymethyl-1-methyl-5-methylaminopyrazole,
- 4-amino-1,3-diphenyl-5-phenylaminopyrazole,
- 4-amino-3-methyl-5-methylamino-1-phenylpyrazole,
- 4-amino-1,3-dimethyl-5-hydrazinopyrazole,
- 5-amino-3-methyl-4-methylamino-1-phenylpyrazole,
- 5-amino-1-methyl-4-(N,N-methylphenyl)amino-3-(4'-chlorophenyl)pyrazole,
- 5-amino-3-ethyl-1-methyl-4-(N,N-methylphenyl)aminopyrazole,
- 5-amino-1-methyl-4-(N,N-methylphenyl)amino-3-phenylpyrazole,
- 5-amino-3-ethyl-4-(N,N-methylphenyl)aminopyrazole,
- 5-amino-4-(N,N-methylphenyl)amino-3-phenylpyrazole,
- 5-amino-4-(N,N-methylphenyl)amino-3-(4'-methylphenyl)-pyrazole,
- 5-amino-3-(4'-chlorophenyl)-4-(N,N-methylphenyl)aminopyrazole,
- 5-amino-3-(4'-methoxyphenyl)-4-(N,N-methylphenyl)-aminopyrazole,
- 4-amino-5-methylamino-3-phenylpyrazole,
- 4-amino-5-ethylamino-3-phenylpyrazole,
- 4-amino-5-ethylamino-3-(4'-methylphenyl)pyrazole,
- 4-amino-3-phenyl-5-propylaminopyrazole,
- 4-amino-5-butylamino-3-phenylpyrazole,
- 4-amino-3-phenyl-5-phenylaminopyrazole,
- 4-amino-5-benzylamino-3-phenylpyrazole,
- 4-amino-5-(4'-chlorophenyl)amino-3-phenylpyrazole,
- 4-amino-3-(4'-chlorophenyl)-5-phenylaminopyrazole,
- 4-amino-3-(4'-methoxyphenyl)-5-phenylaminopyrazole,
- 1-(4'-chlorobenzyl)-4,5-diamino-3-methylpyrazole,
- 4,5-diamino-3-hydroxymethyl-1-isopropylpyrazole,
- 4-amino-1-ethyl-3-methyl-5-methylaminopyrazole,
- 4-amino-5-(2'-aminoethyl)amino-1,3-dimethylpyrazole,
and the addition salts thereof with an acid.

13. Composition according to Claim 12, **characterized in that** the 4,5-diaminopyrazoles of formula (V) are chosen from:
- 4,5-diamino-1,3-dimethylpyrazole,
- 4,5-diamino-3-methyl-1-phenylpyrazole,
- 4,5-diamino-1-methyl-3-phenylpyrazole,
- 4-amino-1,3-dimethyl-5-hydrazinopyrazole,
- 1-benzyl-4,5-diamino-3-methylpyrazole,
- 4,5-diamino-3-tert-butyl-1-methylpyrazole,
- 4,5-diamino-1-tert-butyl-3-methylpyrazole,
- 4,5-diamino-1-(β-hydroxyethyl)-3-methylpyrazole,
- 4,5-diamino-1-ethyl-3-methylpyrazole,
- 4,5-diamino-1-ethyl-3-(4'-methoxyphenyl)pyrazole,
- 4,5-diamino-1-ethyl-3-hydroxymethylpyrazole,
- 4,5-diamino-3-hydroxymethyl-1-methylpyrazole,
- 4,5-diamino-3-hydroxymethyl-1-isopropylpyrazole,
- 4,5-diamino-3-methyl-1-isopropylpyrazole,
- 4-amino-5-(2'-aminoethyl)amino-1,3-dimethylpyrazole,
and the addition salts thereof with an acid.

14. Composition according to Claim 9, **characterized in that** the triaminopyrazoles of formula (VI) are chosen from 3,4,5-triaminopyrazole, 1-methyl-3,4,5-triaminopyrazole, 3, 5-diamino-1-methyl-4-methylaminopyrazole and 3,5-diamino-4-(β-hydroxyethyl)amino-1-methylpyrazole, and the addition salts thereof with an acid.

15. Composition according to any one of the preceding Claims 1 to 14, **characterized in that** the 4,5- or 3,4-diaminopyrazole(s) and/or the triaminopyrazole(s) and/or the corresponding addition salt(s) with an acid represent from 0.0005% to 12% by weight relative to the total weight of the dye composition.

16. Composition according to Claim 15, **characterized in that** the 4,5- or 3,4-diaminopyrazole(s) and/or the triaminopyrazole(s) and/or the corresponding addition salt(s) with an acid represent from 0.005% to 6% by weight relative to the total weight of the dye composition.

17. Composition according to any one of the preceding Claims 1 to 16, **characterized in that** the weight ratio of the carbonyl compound(s) to the 4,5- or 3,4-diaminopyrazole(s) and/or the triaminopyrazole(s) and/or the addition salt(s) with an acid is between 0.001 and 100 and preferably between 0.01 and 10.

18. Composition according to any one of the preceding Claims 1 to 17, **characterized in that** the addition salts with an acid are chosen from the hydrochlorides, hydrobromides, sulfates, tartrates, lactates and acetates.

19. Composition according to any one of the preceding Claims 1 to 18, **characterized in that** it contains at least one coupler.

20. Composition according to Claim 19, **characterized in that** the coupler(s) represent(s) from 0.0001% to 10% by weight approximately relative to the total weight of the dye composition, and even more preferably from 0.005% to 5% by weight approximately relative to this weight.

21. Composition according to any one of the preceding Claims 1 to 20, **characterized in that** it contains at least one additional oxidation base other than the pyrazoles defined in Claims 1 to 14.

22. Composition according to Claim 21, **characterized in that** the additional oxidation base(s) represent(s) from 0.0005% to 12% by weight approximately relative to the total weight of the dye composition.

23. Composition according to any one of the preceding Claims 1 to 22, **characterized in that** it has a pH of between 3 and 12.

24. Composition according to any one of the preceding Claims 1 to 23, **characterized in that** it is in the form of liquids, creams or gels or in any other form that is suitable for dyeing keratin fibres, and especially human hair.

25. Process for dyeing keratin fibres, and in particular human keratin fibres such as the hair, **characterized in that** at least one dye composition as defined in any one of Claims 1 to 24 is applied to these fibres, and the colour is revealed at acidic, neutral or alkaline pH using an oxidizing agent.

26. Process according to Claim 25, **characterized in that** the oxidizing agent present in the oxidizing composition is chosen from hydrogen peroxide, urea peroxide, alkali metal bromates, persalts such as perborates, percarbonates and persulfates, and peracids.

27. Process for dyeing keratin fibres, and in particular human keratin fibres such as the hair, **characterized in that** a composition containing at least one carbonyl compound as defined in any one of Claims 1 to 24 is applied to these fibres in a first stage, a composition containing at least one diaminopyrazole as defined in any one of Claims 1 to 24 is applied in a second stage, the application of the composition containing the carbonyl compound(s) possibly being followed by a rinsing step, the colour being developed using an oxidizing agent.

28. Multi-compartment device comprising a first compartment containing a dye composition as defined in any one of Claims 1 to 24 and a second compartment containing an oxidizing composition.

29. Multi-compartment device comprising a first compartment containing a carbonyl compound as defined in any one of Claims 1 to 24, a second compartment containing a diaminopyrazole as defined in any one of Claims 1 to 24, and a third compartment containing an oxidizing composition.

## Patentansprüche

1. Zusammensetzung zum oxidativen Färben von menschlichen Keratinfasern und insbesondere menschlichen Keratinfasern, wie Haaren, die in einem zum Färben geeigneten Träger enthält:
- mindestens eine Oxidationsbase, die unter den 4,5- oder 3,4-Diaminopyrazolen und den Triaminopyrazolen ausgewählt ist, und
- mindestens eine Carbonylverbindung, die unter den Verbindungen der Formeln (I), (II) und (III) und den Polyimiden ausgewählt ist: 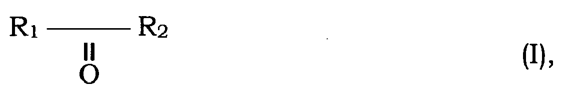 worin bedeuten:
- R₁ und R₂ unabhängig voneinander ein Wasserstoffatom; eine gesättigte oder ungesättigte, aliphatische Kohlenwasserstoffgruppe mit 1 bis 30 Kohlenstoffatomen, die gegebenenfalls durch ein oder mehrere Heteroatome oder eine oder mehrere Carbonylgruppen unterbrochen und unsubstituiert vorliegt oder mit einer oder mehreren Gruppen substituiert ist, die unter Hydroxy, C₁₋₄-Alkoxy, Amino, Carboxy, C₁₋₁₀-Alkoxycarbonyl, Halogen, Nitro, Mono- oder Dialkyl(C₁₋₄)amino, Mono- oder Dihydroxyalkyl(C₁₋₄)amino oder C₆₋₂₀-Aryl ausgewählt sind; eine C₆₋₂₀-Arylgruppe, die gegebenenfalls mit einer oder mehreren Gruppen substituiert ist, die unter Hydroxy, Amino, Nitro, Halogen, Carboxy, Alkoxycarbonyl(C₁₋₁₀), Alkyl(C₁₋₄), Mono- oder Polyhydroxyalkyl(C₁₋₄), Alkoxy(C₁₋₄), Mono- oder Dialkyl (C₁₋₄)amino, Mono- oder Dihydroxyalkyl(C₁₋₄)amino ausgewählt sind;
R₁ und R₂ können gemeinsam mit der Gruppe C=O eine gesät tigten Ring bilden, der gegebenenfalls an einen oder mehrere Benzolringe angebunden sein kann, die mit einer oder mehreren C₁₋₁₀-Alkylgruppen, C₁₋₁₀-Alkoxygruppen, Carboxy oder C₁₋₁₀-Alkoxycarbonylgruppen substituiert sein können; 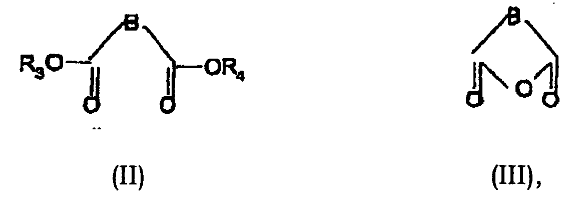
worin bedeuten:
- B eine gesättigte oder ungesättigte, aliphatische Kohlenwasserstoffgruppe mit 1 bis 30 Kohlenstoffatomen, die gegebenenfalls durch ein oder mehrere Heteroatome oder eine oder mehrere Carbonylgruppen unterbrochen und gegebenenfalls mit einer oder mehreren Gruppen substituiert ist, die unter Hydroxy, C₁₋₄-Alkoxy, Amino, Carboxy, C₁₋₁₀-Alkoxycarbonyl, Halogen, Nitro, Mono- oder Dialkyl(C₁₋₄)amino, Mono- oder Di-hydroxyalkyl(C₁₋₄)amino oder C₆₋₂₀-Aryl ausgewählt sind; eine C₆₋₂₀-Arylgruppe, die unsubstituiert ist oder gegebenenfalls mit einer oder mehreren Gruppen substituiert ist, die unter Hydroxy, Amino, Nitro, Halogen, Carboxy, Alkoxycarbonyl(C₁₋₁₀), Alkyl(C₁₋₄), Mono- oder Polyhydroxyalkyl(C₁₋₄), Alkoxy(C₁₋₄), Mono- oder Dialkyl(C₁₋₄)amino, Mono- oder Dihydroxyalkyl(C₁₋₄)amino ausgewählt sind;
- R₃ und R₄, die gleich oder verschieden sind, ein Wasserstoffatom; eine C₁₋₁₀-Alkylgruppe, C₁₋₁₀-Monohydroxyalkyl, C₂₋₁₀₋Polyhydroxyalkyl, Na, K oder NH₄.

2. Zusammensetzung nach Anspruch 1, **dadurch gekennzeichnet, dass** die Verbindungen der Formel (I) unter den aliphatischen Aldehyden oder Ketonen, aromatischen Aldehyden oder Ketonen, β-Dicarbonylverbindungen, γ-Pyronen, Chromonen, Aldosen und Ketosen ausgewählt sind.

3. Zusammensetzung nach Anspruch 1, **dadurch gekennzeichnet, dass** es sich bei der Verbindung der Formel (II) um Maleinsäure handelt.

4. Zusammensetzung nach Anspruch1, **dadurch gekennzeichnet, dass** es sich bei der Verbindung der Formel (III) um Maleinsäureanhydrid handelt.

5. Zusammensetzung nach Anspruch 1, **dadurch gekennzeichnet, dass** das Polyimid ein aromatisches Polyimid ist.

6. Zusammensetzung nach Anspruch 5, **dadurch gekennzeichnet, dass** das Polyimid aus Pyromellitsäuredianhydrid oder Benzophenondianhydrid ausgewählt ist.

7. Zusammensetzung nach einem der Ansprüche 1 bis 6, **dadurch gekennzeichnet, dass** die Carbonylverbindung(en) etwa 0,00001 bis 10 Gew.-% des Gesamtgewichts der Farbmittelzusammensetzung, vorzugsweise 0,001 bis 5 % und noch bevorzugter etwa 0,001 bis 3 Gew.-% des Gesamtgewichts ausmachen.

8. Zusammensetzung nach einem der Ansprüche 1 bis 7, **dadurch gekennzeichnet, dass** die 4,5- oder 3,4-Diaminopyrazole unter den 4,5- oder 3,4-Diaminopyrazolen der folgenden Formeln (IV) oder (V) und/oder deren Additionssalzen mit einer Säure ausgewählt sind: 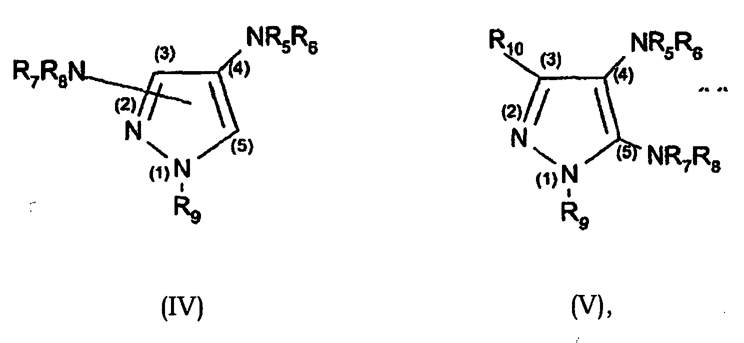 worin bedeuten:
- die Gruppen R₅, R₆, R₇, R₈ und R₉, die gleich oder verschieden sind, ein Wasserstoffatom; eine C₁₋₆-Alkylgruppe, die unsubstituiert vorliegt oder mit mindestens einem Substituenten substituiert ist, der unter OR, NHR, NRR', SR, SOR, SO₂R, COR, COOH, CONH₂, CONHR, CONRR', PO(OH)₂, SH, SO₃X, einem nicht kationischen Heterocyclus, Cl, Br oder I ausgewählt ist, wobei X ein Wasserstoffatom, Na, K oder NH₄ bedeutet und die Gruppen R und R', die gleich oder verschieden sind, eine Alkyl- oder Alkenylgruppe mit 1 bis 4 Kohlenstoffatomen bedeuten; C₂₋₄-Hydroxyalkyl; C₂₋₄-Aminoalkyl; Phenyl; eine Phenylgruppe, die mit einem Halogenatom oder C₁₋₄-Alkyl, C₁₋₄-Alkoxy, Nitro, Trifluormethyl, Amino oder C₁₋₄-Alkylamino substituiert ist; Benzyl; eine Benzylgruppe, die mit einem Halogenatom oder C₁₋₄-Alkyl, C₁₋₄-Alkoxy, Methylendioxy oder Amino substituiert ist; eine Gruppe 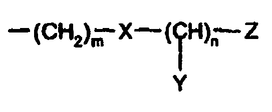 worin m und n, die gleich oder verschieden sind, 0 oder ganze Zahlen von 1 bis 3 bedeuten, X ein Sauerstoffatom oder die Gruppe NH bedeutet, Y ein Wasserstoffatom oder eine C₁₋₄₋Alkylgruppe ist und Z eine Methylgruppe bedeutet, wenn n 0 ist, oder Z eine C₁₋₄-Alkylgruppe, OR oder NR"R"' bedeutet, wenn n 1 bedeutet oder darüber liegt, wobei R" und R"', die gleich oder verschieden sind, ein Wasserstoffatom oder C₁₋₄₋Alkyl bedeuten; oder R₉ bildet mit dem Stickstoffatom der Gruppe NR₇R₈ in 5-Stellung einen Heterocyclus, der mindestens 4 Ringatome aufweist;
- Rio C₁₋₆-Alkyl; C₁₋₄-Hydroxyalkyl; C₁₋₄-Aminoalkyl; Alkyl(C₁₋₄)-aminoalkyl(C₁₋₄); Dialkyl(C₁₋₄)aminoalkyl(C₁₋₄); Hydroxyalkyl(C₁₋₄)aminoalkyl(C₁₋₄); Alkoxy(C₁₋₄)methyl; Phenyl; eine Phenylgruppe, die mit einem Halogenatom oder Alkyl(C₁₋₄), Alkoxy(C₁₋₄), Nitro, Trifluormethyl, Amino oder Alkyl(C₁₋₄)-amino substituiert ist; Benzyl; eine Benzylgruppe, die mit einem Halogenatom oder Alkyl(C₁₋₄), Alkoxy(C₁₋₄), Nitro, Trifluormethyl, Amino oder Alkyl(C₁₋₄)amino substituiert ist; einen Heterocyclus, der unter Thiophen, Furan und Pyridin ausgewählt ist, oder eine Gruppe -(CH₂)ₚ-O-(CH₂)_{q}-OR", wobei p und q, die gleich oder voneinander verschieden sind, ganze Zahlen im Bereich von 1 bis 3 bedeuten und R" die oben angegebenen Bedeutungen aufweist,
mit der Maßgabe, dass:
mindestens eine der Gruppen R₅, R₆, R₇ und R₈ ein Wasserstoffatom bedeutet.

9. Zusammensetzung nach einem der Ansprüche 1 bis 7, **dadurch gekennzeichnet, dass** die Triaminopyrazole unter den Verbindungen der folgenden Formel (VI) und/oder den Additionssalzen mit einer Säure ausgewählt sind: 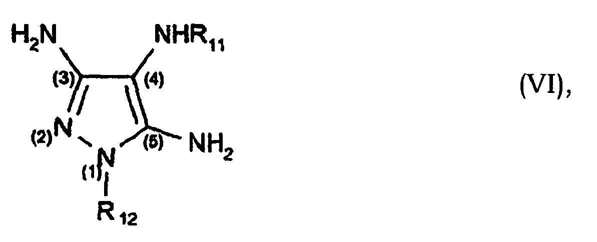 worin bedeuten:
- R₁₁ und R₁₂, die gleich oder verschieden sind, ein Wasserstoffatom, C₁₋₄-Alkyl oder C₂₋₄-Hydroxyalkyl.

10. Zusammensetzung nach Anspruch 8, **dadurch gekennzeichnet, dass** die 4,5- oder 3,4-Diaminopyrazole der Formel (IV) ausgewählt sind unter: 4,5-Diamino-1-(4'-methoxybenzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-(4'-methoxybenzyl)-5-methylamino-pyrazol, 4-Amino-5-(β-hydroxyethyl)-amino-1-(4'-methoxybenzyl)-pyrazol, 4-Amino-5-(β-hydroxyethyl)-amino-1-methyl-pyrazol, 4-Amino-(3)5-methylamino-pyrazol, 3(5),4-Diamino-pyrazol, 4,5-Diamino-1-methyl-pyrazol, 4,5-Diamino-1-ethyl-pyrazol, 4,5-Diamino-1-isopropyl-pyrazol, 4,5-Diamino-1-hydroxyethyl-pyrazol, 4,5-Diamino-1-benzyl-pyrazol, 4-Diamino-5-hydroxyethylamino-1-hydroxyethylpyrazol, 4-Diamino-5-methylamino-1-hydroxyethyl-pyrazol, 3-Amino-4,5,7,8-tetrahydro-pyrazolo[1,5-a]pyrimidin, 7-Amino-2,3-dihydro-1H-imidazol[1,2-b]pyrazol, 3-Amino-8-methyl-4,5,7-8-tetrahydropyrazolo[1,5-a]pyrimidin, 2-(4,5-Diamino-pyrazol-1-yl)-1-ethan-sulfonsäure, 2-(4,5-Diamino-pyrazol-1-yl)-acetamid, 2-(4,5-Diamino-pyrazol-1-yl)-essigsäure, 2-(2-Dimethylaminoethyl)-2H-pyrazol-3,4-diamin, 2-(2-Methoxy-ethyl)-2H-pyrazol-3,4-diamin und deren Additionssalze mit einer Säure.

11. Zusammensetzung nach Anspruch 10, **dadurch gekennzeichnet, dass** die 4,5- oder 3,4-Diaminopyrazole der Formel (IV) ausgewählt sind unter:
- 4,5-Diamino-1-benzyl-pyrazol,
- 4,5-Diamino-1-(4'-chlorbenzyl)-pyrazol,
- 4,5-Diamino-1-methyl-pyrazol,
- 4,5-Diamino-1-hydroxyethyl-pyrazol,
- 2-(2-Methoxy-ethyl)-2H-pyrazol-3,4-diamin
- und deren Additionssalzen mit einer Säure.

12. Zusammensetzung nach Anspruch 8, **dadurch gekennzeichnet, dass** die 4,5-Diaminopyrazole der Formel (V) ausgewählt sind unter:
- 1-Benzyl-4,5-diamino-3-methyl-pyrazol,
- 4,5-Diamino-1-(β-hydroxyethyl)-3-(4'-methoxyphenyl)-pyrazol,
- 4,5-Diamino-1-(β-hydroxyethyl)-3-(4'-methylphenyl)-pyrazol,
- 4,5-Diamino-1-(β-hydroxyethyl)-3-(β-methylphenyl)-pyrazol,
- 4,5-Diamino-3-methyl-1-isopropyl-pyrazol,
- 4,5-Diamino-3-(4'-methoxyphenyl)-1-isopropyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-3-hydroxymethyl-1-methyl-pyrazol,
- 4,5-Diamino-1-ethyl-3-hydroxymethyl-pyrazol,
- 4,5-Diamino-3-hydroxymethyl-1-isopropyl-pyrazol,
- 4,5-Diamino-3-hydroxymethyl-1-*t*-butyl-pyrazol,
- 4,5-Diamino-3-hydroxymethyl-1-phenyl-pyrazol,
- 4,5-Diamino-3-hydroxymethyl-1-(2'-methoxyphenyl)-pyrazol,
- 4,5-Diamino-3-hydroxymethyl-1-(3'-methoxyphenyl)-pyrazol,
- 4,5-Diamino-3-hydroxymethyl-1-(4'-methoxyphenyl)-pyrazol,
- 1-Benzyl-4,5-diamino-3-hydroxymethyl-pyrazol,
- 4,5-Diamino-3-methyl-1-(2'-methoxyphenyl)-pyrazol,
- 4,5-Diamino-3-methyl-1-(3'-methoxyphenyl)-pyrazol,
- 4,5-Diamino-3-methyl-1-(4'-methoxyphenyl)-pyrazol,
- 3-Aminomethyl-4,5-diamino-1-methyl-pyrazol,
- 3-Aminomethyl-4,5-diamino-1-ethyl-pyrazol,
- 3-Aminomethyl-4,5-diamino-1-isopropyl-pyrazol,
- 3-Aminomethyl-4,5-diamino-1-*t*-butyl-pyrazol,
- 4, 5-Diamino-3-dimethylaminomethyl-1-methyl-pyrazol,
- 4, 5-Diamino-3-dimethylaminomethyl-1-ethyl-pyrazol,
- 4,5-Diamino-3-dimethylaminomethyl-1-isopropyl-pyrazol,
- 4,5-Diamino-3-dimethylaminomethyl-1-*t*-butyl-pyrazol,
- 4, 5-Diamino-3-ethylaminomethyl-1-methyl-pyrazol,
- 4, 5-Diamino-3-ethylaminomethyl-1-ethyl-pyrazol,
- 4,5-Diamino-3-ethylaminomethyl-1-isopropyl-pyrazol,
- 4,5-Diamino-3-ethylaminomethyl-1-*t*-butyl-pyrazol,
- 4,5-Diamino-3-methylaminomethyl-1 -methyl-pyrazol,
- 4,5-Diamino-3-methylaminomethyl-1-isopropyl-pyrazol,
- 4,5-Diamino-1-ethyl-3-methylaminomethyl-pyrazol,
- 1-*t*-Butyl-4,5-diamino-3-methylaminomethyl-pyrazol,
- 4,5-Diamino-3-[(β-hydroxyethyl)aminomethyl]- 1-methyl-pyrazol,
- 4,5-Diamino-3-[(β-hydroxyethyl)aminomethyl]-1-isopropyl-pyrazol,
- 4,5-Diamino-1-ethyl-3-[(β-hydroxyethyl)aminomethyl]-pyrazol,
- 1-*t*-Butyl-4,5-diamino-3-[(β-hydroxyethyl)aminomethyl]-pyrazol,
- 4-Amino-5-(β-hydroxyethyl)amino-1,3-dimethyl-pyrazol,
- 4-Amino-5-(β-hydroxyethyl)amino-1-isopropyl-3-methyl-pyrazol,
- 4-Amino-5-(β-hydroxyethyl)amino-1-ethyl-3-methyl-pyrazol,
- 4-Amino-5-(β-hydroxyethyl)amino-1-*t*-butyl-3-methyl-pyrazol,
- 4-Amino-5-(β-hydroxyethyl)amino-1-phenyl-3-methyl-pyrazol,
- 4-Amino-5-(β-hydroxyethyl)amino-1-(2-methoxyphenyl)-3-methyl-pyrazol,
- 4-Amino-5-(β-hydroxyethyl)amino-1-(3-methoxyphenyl)-3-methyl-pyrazol,
- 4-Amino-5-(β-hydroxyethyl)amino-1-(4-methoxyphenyl)-3-methyl-pyrazol,
- 4-Amino-5-(β-hydroxyethyl)amino-1-benzyl-3-methyl-pyrazol,
- 4-Amino-1-ethyl-3-methyl-5-methylamino-pyrazol,
- 4-Amino-1-*t*-butyl-3-methyl-5-methylamino-pyrazol,
- 4,5-Diamino-1,3-dimethyl-pyrazol,
- 4,5-Diamino-3-*t*-butyl-1-methyl-pyrazol,
- 4,5-Diamino-1-*t*-butyl-3-methyl-pyrazol,
- 4,5-Diamino-1-methyl-3-phenyl-pyrazol,
- 4,5-Diamino-1-(β-hydroxyethyl)-3-methyl-pyrazol,
- 4,5-Diamino-1-(β-hydroxyethyl)-3-phenyl-pyrazol,
- 4,5-Diamino-1-methyl-3-(2'-chlorphenyl)-pyrazol,
- 4,5-Diamino-1-methyl-3-(4'-chlorphenyl)-pyrazol,
- 4,5-Diamino-1-methyl-3-(3'-trifluormethylphenyl)-pyrazol,
- 4,5-Diamino-1,3-diphenyl-pyrazol,
- 4,5-Diamino-3-methyl-1-phenyl-pyrazol,
- 4-Amino-1,3-dimethyl-5-phenylamino-pyrazol,
- 4-Amino-1-ethyl-3-methyl-5-phenylamino-pyrazol,
- 4-Amino-1,3-dimethyl-5-methylamino-pyrazol,
- 4-Amino-3-methyl-1-isopropyl-5-methylamino-pyrazol,
- 4-Amino-3-isobutoxymethyl-1-methyl-5-methylamino-pyrazol,
- 4-Amino-3-methoxyethoxymethyl-1-methyl-5-methylaminopyrazol,
- 4-Amino-3-hydroxymethyl-1-methyl-5-methylamino-pyrazol,
- 4-Amino-1,3-diphenyl-5-phenylamino-pyrazol,
- 4-Amino-3-methyl-5-methylamino-1-phenyl-pyrazol,
- 4-Amino-1,3-dimethyl-5-hydrazino-pyrazol,
- 5-Amino-3-methyl-4-methylamino-1-phenyl-pyrazol,
- 5-Amino-1-methyl-4-(N,N-methylphenyl)amino-3-(4'-chlorphenyl)-pyrazol,
- 5-Amino-3-ethyl-1-methyl-4-(N,N-methylphenyl)aminopyrazol,
- 5-Amino-1-methyl-4-(N,N-methylphenyl)amino-3-phenylpyrazol,
- 5-Amino-3-ethyl-4-(N,N-methylphenyl)amino-pyrazol,
- 5-Amino-4-(N,N-methylphenyl)amino-3-phenyl-pyrazol,
- 5-Amino-4-(N,N-methylphenyl)amino-3-(4'-methylphenyl)-pyrazol,
- 5-Amino-3-(4'-chlorphenyl)-4-(N,N-methylphenyl)aminopyrazol,
- 5-Amino-3-(4'-methoxyphenyl)-4-(N,N-methylphenyl)aminopyrazol,
- 4-Amino-5-methylamino-3-phenyl-pyrazol,
- 4-Amino-5-ethylamino-3-phenyl-pyrazol,
- 4-Amino-5-ethylamino-3-(4'-methylphenyl)-pyrazol,
- 4-Amino-3-phenyl-5-propylamino-pyrazol,
- 4-Amino-5-butylamino-3-phenyl-pyrazol,
- 4-Amino-3-phenyl-5-phenylamino-pyrazol,
- 4-Amino-5-benzylamino-3-phenyl-pyrazol,
- 4-Amino-5-(4'-chlorphenyl)-amino-3-phenyl-pyrazol,
- 4-Amino-3-(4'-chlorphenyl)-5-phenylamino-pyrazol,
- 4-Amino-5-(4'-methoxyphenyl)-5-phenylamino-pyrazol,
- 1-(4'-Chlorbenzyl)-4,5-diamino-3-methyl-pyrazol,
- 4,5-Diamino-3-hydroxymethyl- 1-isopropyl-pyrazol,
- 4-Amino-1-ethyl-3-methyl-5-methylamino-pyrazol,
- 4-Amino-5-(2'-aminoethyl)amino-1,3-dimethyl-pyrazol
- und deren Additionssalzen mit einer Säure.

13. Zusammensetzung nach Anspruch 12, **dadurch gekennzeichnet, dass** die 4,5-Diaminopyrazole der Formel (V) ausgewählt sind unter:
- 4,5-Diamino-1,3-dimethyl-pyrazol,
- 4,5-Diamino-3-methyl-1-phenyl-pyrazol,
- 4,5-Diamino-1-methyl-3-phenyl-pyrazol,
- 4-Amino-1,3-dimethyl-5-hydrazino-pyrazol,
- 1-Benzyl-4,5-diamino-3-methyl-pyrazol,
- 4,5-Diamino-3-*t*-butyl-1-methyl-pyrazol,
- 4,5-Diamino-1-*t*-butyl-3-methyl-pyrazol,
- 4,5-Diamino-1-(β-hydroxyethyl)-3-methyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-1-ethyl-3-hydroxymethyl-pyrazol,
- 4,5-Diamino-3-hydroxymethyl-1-methyl-pyrazol,
- 4,5-Diamino-3-hydroxymethyl-1-isopropyl-pyrazol,
- 4,5-Diamino-3-methyl-1-isopropyl-pyrazol,
- 4-Amino-5-(2'-aminoethyl)-amino-1,3-dimethyl-pyrazol
- und deren Additionssalzen mit einer Säure.

14. Zusammensetzung nach Anspruch 9, **dadurch gekennzeichnet, dass** die Triaminopyrazole der Formel (VI) unter 3,4,5-Triaminopyrazol, 1-Methyl-3,4,5-triamino-pyrazol, 3,5-Diamino-1-methyl-4-methylamino-pyrazol und 3, 5-Diamino-4-(β-hydroxyethyl)-amino-1-methyl-pyrazol und deren Additionssalze mit einer Säure ausgewählt sind.

15. Zusammensetzung nach einem der vorhergehenden Ansprüche 1 bis 14, **dadurch gekennzeichnet, dass** das oder die 4,5- oder 3,4-Diaminopyrazol(e) und/oder das oder die Triaminopyrazol(e) und/oder das oder die entsprechende(n) Additionssalz(e) mit einer Säure 0,0005 bis 12 Gew.-% des Gesamtgewichts der Farbmittelzusammensetzung ausmachen.

16. Zusammensetzung nach Anspruch 15, **dadurch gekennzeichnet, dass** das oder die 4,5- oder 3,4-Diaminopyrazol(e) und/oder das oder die Triaminopyrazol(e) und/oder das oder die entsprechende(n) Additionssalz(e) mit einer Säure 0,005 bis 6 Gew.-% des Gesamtgewichts der Farbmittelzusammensetzung ausmachen.

17. Zusammensetzung nach einem der Ansprüche 1 bis 16, **dadurch gekennzeichnet, dass** das Gewichtsverhältnis der Carbonylverbindung(en) und des oder der 4,5- oder 3,4-Diaminopyrazole und/oder des oder der Triaminopyrazole und/oder des oder der Additionssalze mit einer Säure im Bereich von 0,001 bis 100 und vorzugsweise 0,01 bis 10 liegt.

18. Zusammensetzung nach einem der vorhergehenden Ansprüche 1 bis 17, **dadurch gekennzeichnet, dass** die Additionssalze mit einer Säure unter den Hydrochloriden, Hydrobromiden, Sulfaten, Tartraten, Lactaten und Acetaten ausgewählt sind.

19. Zusammensetzung nach einem der vorhergehenden Ansprüche 1 bis 18, **dadurch gekennzeichnet, dass** sie mindestens einen Kuppler enthält.

20. Zusammensetzung nach Anspruch 19, **dadurch gekennzeichnet, dass** der oder die Kuppler etwa 0,0001 bis 10 Gew.-% des Gesamtgewichts der Farbmittelzusammensetzung und noch bevorzugter etwa 0,005 bis 5 Gew.-% des Gesamtgewichts ausmachen.

21. Zusammensetzung nach einem der vorhergehenden Ansprüche 1 bis 20, **dadurch gekennzeichnet, dass** sie mindestens eine zusätzliche Oxidationsbase enthält, die von dem in den Ansprüchen 1 bis 14 definierten Pyrazolen verschieden ist.

22. Zusammensetzung nach Anspruch 21, **dadurch gekennzeichnet, dass** die zusätzliche(n) Oxidationsbase(n) etwa 0,0005 bis 12 Gew.-% des Gesamtgewichts der Farbmittelzusammensetzung ausmachen.

23. Zusammensetzung nach einem der vorhergehenden Ansprüche 1 bis 22, **dadurch gekennzeichnet, dass** sie einen pH-Wert im Bereich von 3 bis 12 aufweist.

24. Zusammensetzung nach einem der vorhergehenden Ansprüche 1 bis 23, **dadurch gekennzeichnet, dass** sie als Flüssigkeit, Creme oder Gel oder beliebigen anderen Formen vorliegt, die geeignet sind, Keratinfasern und insbesondere menschliches Haar zu färben.

25. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 24 aufgetragen wird und die Farbe bei einem sauren, neutralen oder alkalischen pH-Wert mit einem Oxidationsmittel entwickelt wird.

26. Verfahren nach Anspruch 25, **dadurch gekennzeichnet, dass** das Oxidationsmittel, das in der oxidierenden Zusammensetzung vorliegt, unter Wasserstoffperoxid, Harnstoffperoxid, Alkalimetallbromaten, Salzen von Persäuren, wie Perboraten, Percarbonaten und Persulfaten, und Persäuren ausgewählt ist.

27. Verfahren zum Färben von Keratinfasern und insbesondere menschlichen Keratinfasern wie Haaren, **dadurch gekennzeichnet, dass** auf die Fasern in einem ersten Schritt eine Zusammensetzung, die mindestens eine Carbonylverbindung enthält, wie in einem der Ansprüche 1 bis 24 definiert, und in einem zweiten Schritt eine Zusammensetzung aufgebracht wird, die mindestens ein Diaminopyrazol enthält, wie in einem der Ansprüche 1 bis 24 definiert, wobei nach dem Aufbringen der Zusammensetzung, die die Carbonylverbindung(en) enthält, gegebenenfalls gespült wird und wobei die Farbe mit einem Oxidationsmittel entwickelt wird.

28. Vorrichtung mit mehreren Abteilungen, die eine erste Abteilung, die eine Farbmittelzusammensetzung nach einem der Ansprüche 1 bis 24 enthält, und eine zweite Abteilung aufweist, die eine oxidierende Zusammensetzung enthält.

29. Vorrichtung mit mehreren Abteilungen, die eine erste Abteilung mit einer Carbonylverbindung, wie in einem der Ansprüche 1 bis 24 definiert, eine zweite Abteilung mit einem Diaminopyrazol, wie in einem der Ansprüche 1 bis 24 definiert, und eine dritte Abteilung mit einer oxidierenden Zusammensetzung enthält.
